(19)

Europäisches
Patentamt
European
Patent Office
Office européen
des brevets

(11) **EP 2 494 957 A1**

(12) **EUROPEAN PATENT APPLICATION**
published in accordance with Art. 153(4) EPC

(43) Date of publication:
**05.09.2012 Bulletin 2012/36**

(51) Int Cl.:
*A61K 9/107* (2006.01)　　*A61K 31/337* (2006.01)
*A61P 35/00* (2006.01)

(21) Application number: **10826102.5**

(22) Date of filing: **28.10.2010**

(86) International application number:
**PCT/CN2010/078209**

(87) International publication number:
**WO 2011/050739 (05.05.2011 Gazette 2011/18)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.10.2009　CN 200910236959**

(71) Applicant: **Institute of Mataria Medica, Chinese
Academy
of Medical Sciences
Beijing 100050 (CN)**

(72) Inventors:
 • **LIU, Yuling
  Beijing 100050 (CN)**
 • **Xia, Xuejun
  Beijing 100050 (CN)**
 • **GUO, Ruifang
  Beijing 100050 (CN)**

 • **ZHANG, Pengxiao
  Beijing 100050 (CN)**
 • **HAN, Rui
  Beijing 100050 (CN)**
 • **FU, Zhaodi
  Beijing 100050 (CN)**
 • **ZHOU, Cuiping
  Beijing 100050 (CN)**
 • **WANG, Renyun
  Beijing 100050 (CN)**
 • **JIN, Dujia
  Beijing 100050 (CN)**

(74) Representative: **Hart-Davis, Jason et al
Cabinet Beau de Loménie
158, rue de l'Université
75340 Paris Cedex 07 (FR)**

(54) **SUBMICRO EMULSION OF PACLITAXEL USING STEROID COMPLEX AS INTERMEDIATE CARRIER**

(57)　　A submicron emulsion of paclitaxel, the preparation method and the use thereof are disclosed. Said paclitaxel submicron emulsion comprises paclitarxel/steroid complex, oil for injection, water for injection, emulsifier, assistant emulsifier and isotonic agent, wherein the mole ratio of paclitaxel to steroid in the complex is 1: 0.2~4; preferably 1:0.25-2. Said submicron emulsion is useful for the treatment for malignant tumor. The average particle diameter of the submicron emulsion is less than 400 nm and the pH Value is 3.5-6.

EP 2 494 957 A1

**Description**

**Field of the Invention**

The present invention relates to a paclitaxel submicron emulsion using steroid complex as intermediate carrier and its preparation procedure, and also the application of this paclitaxel submicron emulsion, which belongs to the field of pharmaceutical preparation technology.

**Background of the Invention**

Paclitaxel (paditaxel, Taxol) possesses an important anti-tumor activity, thus has been widely used in the treatment of ovarian and breast cancer, non-small cell lung cancer (NSCLC), head and neck carcinoma in clinical practice. Since it is barely soluble in water (0.006 $\mu$g/ml), common paclitaxel injection Taxol® presently used in clinical practice is prepared through dissolving 30 mg paclitaxel into 5 ml mixture of Cremopher EL (ethoxylate castor oil)/ alcohol (50: 50, V/V). Because of the large amount of Cremopher EL in the formulation, it tends to stimulate the release of histamine in vivo, resulting in severe allergic reactions. With regard to this, the following desensitization is to be done before clinical use: 10 mg oral dexamethasone is given 12 hours before treatment, and a second dose of 10 mg oral dexamethasone is used 6 hours before treatment, and 30 to 60 minutes before treatment, diphenhydramine, 20 mg, i.m., cimetidine, 300 mg, i.v. or ranitidine, 50 mg, i.v. are given. Even though, 5% to 30% of the patient may experience allergies at different degrees in clinical practice. Furthermore, there may be physical stability issue once the concentrated paclitaxel solution solubilized by Cremopher EL/alcohol is diluted, for example, the drug may precipitate due to a low temperature or a long instilling time, thus patients' safety may be at risk.

Against the undesired effect of paclitaxel injection, pharmaceuticals both in China and worldwide has performed studies on a new release system since paclitaxel was launched more than 20 years ago, and technological strategies include cyclodextrin inclusion complex, liposome, polymeric micelle, nanoparticle and so on.

Although dextrin inclusion complex could increase paclitaxel's solubility, yet too much cyclodextrin used is subject to cause severe renal toxicity, also the drug may precipitate once dilution is performed through water, therefore, it has not been implemented in clinical practice so far.

Liposome has disadvantages including low entrapment efficiency, being prone to leakage if stored for a long time and precipitation after dilution through water, thus it is difficult to develop it commercially and no product of this category has been available even though there has been investment going on abroad for 20 years. The paclitaxel liposome (lipusu) freeze-dried power for inject include 30 mg of the drug in each, its specification and dose for clinical use is identical to common injections available, and the efficacy is not significantly different, however, a preparing procedure is introduced and a pretreatment for desensitization is also needed, therefore, it is not technologically superior.

There are plenty of studies concerning paclitaxel polymeric micelle going on both domestically and abroad, but its industrialization is limited by low drug loading, unstable quality after storage and requirement for lyophilization during storage. In the past years, the research method for polymeric micelle is developing very fast due to the sprouting of new polymer materials, however the medicinal safety of introducing large amount of polymeric materials with completely new structure needs further evaluation.

The protein-bound paclitaxel nanoparticle injection (manufactured by Bioscience, inc.) approved by FDA in 2005 is so far the most important new patent paclitaxel formulation all over the world, and it is designed to use human plasma albumin as a carrier and to prepare the paclitaxel as protein-bound nanoparticle, which is made into freeze-dried power for injection after aspetic filtration, freezing and drying. Compared to common injections, the albumin-bound paclitaxel nanoparticle for injection is superior on the following aspects: 1) this formulation is Cremopher EL free, thus allergic reaction is completely avoided, which makes it the only new paclitaxel formulation requiring no desensitization treatment worldwide; 2) due to its low toxicity and high tolerance, the clinical used dosage for patients is increased from 135~175 mg(m$^2$ to 260 mg/m$^2$, thus resulting in a significantly better clinical efficacy than common paclitaxel injections. However, due to large amount of carrier albumin, which is extremely expensive (up to 6200 Yuan for each injection), as well as highly complicated and strict preparing procedures, the clinical use of albumin-bound paclitaxel nanoparticle is very limited.

The oil-in-water submicron emulsion is a emulsion of particles with an average diameter less than 600 nm obtained through homogeneous emulsification under high pressure using natural phospholipid as emulsifier after dissolving the medicine into the oil phase and the basis is the drugs lipotropy. Because inside the medicine exists inner oil phase, which avoid direct contact between water and air, thus overcoming the disadvantage that slightly soluble medicine is difficult to be prepared into liquid formulation due to low solubility and stability. Compared to liposome technology, submicron emulsion is more convenient to industrialize; and compared to albumin bound nanoparticle, liposome submicron emulsion has a lower manufacturing cost, could be sterilized at terminal, is to be injected directly in clinical practice, does not tend to precipitate, as well as safe and convenient to administrate. Therefore, there is a promising future to develop new paclitaxel formulation using submicron emulsion as carrier. Although both domestic and oversee scholars have done lots of experiments on paclitaxel submicron emulsion, yet the drug loading in the submicron emulsion manufactured through usual procedure is under 0.02 mg/ml due to paclitaxel's slight solubility in the water as well as extremely low solubility in oil, moreover, the medicine may transfer from the oil phase into the water phase during disinfection and storage, resulting in demulsification, stratifying and concentration. Restricted by its low solubility in water, yet no paclitaxel submicron emulsion with high drug loading, tolerating sterilization under heat and pressure and stable quality through long-term storage has been developed worldwide.

In order to improve paclitaxel's solubility in oil phase and ease the restriction from its physicochemical properties on the development of submicron emulsion, we performed preliminary studies on "Paclitaxel Liposome Complex" and "Paclitaxel Submicron Emulsion Using Liposome as Intermediate Carrier", and applied for two patents: application No. 200810168213.X, "Paclitaxel Liposome Complex" and application No. 200810168212.5, "Paclitaxel Submicron Emulsion Using Liposome as Intermediate Carrier".

For the paclitaxel liposome complex disclosed in patent application 200810168213.X, natural egg yolk lecithin, granulesten and cholesterol were carefully chosen as the liposome material, and the proportion of paclitaxel and liposome material is 1:1~19 by weight, i.e. the amount of liposome is up to 1~19 times of that of paclitaxel (more specifically, for phospholipid, the mole ratio between paclitaxel and liposome is 1:1~20; for cholesterol, it is 1:22~20; for bile acids, it is 1:2.1~40). While the submicron emulsion formulation disclosed in patent application 200810168212.5 adopts the paclitaxel liposome complex in patent application 200810168213.X as the intermediate carrier.

The paclitaxel liposome complex is designed to improve the solubility of paclitaxel in oil and provide qualified intermediate carrier for consequential manufacture of submicron emulsion. However, through further investigation, the following flaws are identified in the technological protocol mentioned in patent applications 200810168213.X and 200810168212.5.

1. Although the drug solubility in oil could be significantly improved by using phospholipid to prepare the complex, however, the maximum is limited to 2 mg/ml, and the solubility in oil is not to be further increased by adding more phospholipid; Limited by low solubility in oil, the maximum drug loading is restricted to 0.5 mg/ml if submicron emulsion is prepared by using liposome complex as the intermediate carrier, and the entrapment efficiency is under 80%, there is obvious stratification after storage up to 6 months, thus it could not meet the requirements of medical treatment; with a drug loading up to 1.0 mg/ml, it could not form even emulsion.

2. Cholesterol could significantly better improve the drug solubility in oil than phospholipid when used as the liposome material for the complex, however, cholesterol is a steroid, which could result in various disadvantages since its amount is 1~19 times of that of paclitaxel: **(1) overdose:** a health adult intakes about 300 mg ~ 500 mg cholesterol each day (equivalent to the cholesterol in 1~2 eggs), and one medicinal dose of paclitaxel is 300 mg, as for the cholesterol complex and its formulation involved in patent patent application 200810168213.X, the cholesterol intake is about 300 mg ~ 5700 mg, with the highest dosage equivalent up to 19 egg yolks, which is significantly excess and could pertain to safety risk; (2) **instability of the submicron emulsion prepared through long-term storage:** if cholesterol complex is used as the intermediate carrier during submicron emulsion preparation, based on the medicinal formulation and specific paclitaxel concentration, higher the liposome material is used in the complex, more complex will be wrapped inside the inner oil phase in the submicron emulsion. Restricted by the volume of the oil drop inside the oil phase and interface between oil and water, when the amount of complexes wrapped exceeds that content that the oil phase and the interface between oil and water, part of the drug may be driven to the outer water phase, resulting in a low entrapment efficiency and instability of the submicron emulsion prepared. Through investigation on the submicron emulsion described in patent application 200810168212.5, the entrapment efficiency is 65% to 85%, and the quality is essential stable if stored for 6 months at 4°C, however, there is obvious stratification when it is stored up to 12 months, the declared content drops and the paclitaxel impurity is significantly increased; (3) **high manufacture cost:** factors including large amount of liposome used, solvent largely used during manufacture and long duration taken to volatilize the solvent, result in high manufacture cost, which disobey the principle of pharmacological economy.

For the purpose of providing submicron emulsion with low liposome material, high entrapment efficiency and good stability, after variety of experiments, this submission prepare submicron emulsion using a steroid complex with low liposome material as intermediate carrier. In the steroid complex, the mole ratio of paclitaxel/cholesterol is 1:0.2~4, preferably 1:0.25~2, best 1:0.33~1; correspondingly, the ratio between paclitaxel and cholesterol by weight is 1:0.09~1: 1.94, preferably is 1:0.11~1:0.97, and best 1:0.15~1:0.49. Compared to patent application 200810168212.5, the complex in this application manages a complete paclitaxel combining while significantly reducing the use of steroid, thus increasing the solubility of drug in the oil as it possibly can. The solubility tends to remain stably while the steroid is further increased, indicating no further increasing effect. Since the steroid complex could improve the solubility of the drug in the oil, the complex is dissolved into the oil phase and a paclitaxel submicron emulsion with high entrapment efficiency, stable quality through long-term storage and low cholesterol is obtained using emulsifier and assistant emulsifier, thus the present invention is completed.

## Contents of the Invention

One purpose of the present invention is to provide a paclitaxel submicron emulsion, which consists of paclitaxel/steroid complex, oil for injection, emulsifier, assistant emulsifier and isotonic agent; the steroid in the paclitaxel/steroid complex as described is natural steroid or one of its derivatives; the natural steroid as described is selected from the group consisting of cholesterol, 7-hydrocholesterol (also named 7-dehydrocholesterol), lanosterol, sitosterol, brassicasterol, mycosterol, ostreasterol, stigmasterol, sitosterolum and ergosterol, preferably cholesterol, 7-hydrocholesterol and ergosterol, the best cholesterol; the natural steroid derivatives as described are choosed from cholic acid, deoxycholic acid and anthropodesoxycholic acid.

The mean diameter of the submicron emulsion droplet in the present invention is under 400 nm, preferably under 300 nm; the ratio of oil phase is 5%~35% (ml/ml) total amount of the submicron emulsion as described, preferably 10%~30% (ml/ml); measured by paclitaxel, the drug load is 0.25 mg/ml~5mg/ml, preferably 0.5 mg/ml~2mg/ml.

In the present invention, the paclitaxel/steroid complex as described is prepared through the following procedure 1 or 2, procedure 1 including:

   a. Mix paclitaxel and steroid at specific ratio, add appropriate volume of organic solvent, choose any antioxidative stabilizer and add in;
   b. Agitate under proper temperature, remove the organic solvent through rotating evaporation or sponging drying, and after vacuum drying, the complex is obtained;

Procedure 2 including:

   a. Prepare paclitaxel and steroid with specific ratio, add appropriate volume of different organic solvents respectively, and then mix them, choose any antioxidative stabilizer and add in;
   b. Agitate under proper temperature, remove the organic solvent through rotating evaporation or sponging drying, and after vacuum drying, the complex is obtained.

In the paclitaxel/steroid complex and its preparing procedures as described above, the mole ratio between paclitaxel and steroid is 1:0.2~4, preferably 1:0.25~2, the best 1:0.33~1; correspondingly, the ratio between paclitaxel and steroid by weight is 1:0.09~1:1.94, preferably 1:0.11~1:0.97, and the best is 1:0.15~1:0.49.

In the preparing procedures of paclitaxel/steroid as described above, the organic solvent as described is selected from the group consisting of methylene dichloride, alcohol, methanol, phenylcarbinol, acetone, ethyl acetate, tetrahydrofuran, tertiary butyl alcohol; for preferably, one or more is selected from the group consisting of alcohol, acetone, ethyl acetate and tetrahydrofuran. By "appropriate volume of organic solvent", the "appropriate volume" means the volume that the technician in this filed could determine based on common specification for dissolving the mixture of paclitaxel and steroid, to be specific, the concentration of paclitaxel and steroid complex in the solution calculated by paclitaxel should be controlled to 0.5~16 mg/ml or higher, preferably is 1.0~8.0 mg/ml; "proper temperature" refers to 10°C-70°C, preferably 35-55°C, for example 5°C, 35°C, 45°C, 55°C or 70°C. Both the duration of agitation reaction and vacuum drying could be determined by technicians in this area based on common specification, for example, the time for agitation reaction could be 0.5-3.0 hours, such as 0.5, 1.0, 1.5 or 2.0 hours, and the time for vacuum drying could be 8-48 hours, such as 8, 12, 16 or 24 hours. The antioxidative stabilizer as described is selected from the group consisting of sodium bisulfite, sodium metabisulfite, vitamin C, EDTA and its salt, vitamin E and its derivatives, and the amount of antioxidative stabilizer is the common amount used during the preparation of liposome complex in this area, and it usually does not exceed 1 % (by weight) of the total amount of the complex.

In the present invention, the oil for injection as described is one or mixture from long chain or medium chain oil. The long chain oil as describedis selected from the group consisting of long chain fatty acid, long chain fatty ester or long chain fatty alcohol, to be specific, one selected from the group consisting of soybean oil, castor oil, linoleic acid, maize oil, olive oil, oil of groundnuts, cotton seed oil, oleic acid, glyceryl monostearate, glycerol monooleate, cetanol; the medium chain oil is one selected from the group consisting of medium chain fatty acid and medium chain fatty ester. The preferably for long chain oil is long chain fatty ester, specifically soybean oil for injection; the optimal selection for medium chain oil is long chain fatty acid glyceride.

In the present invention, the emulsifier as described is nonionic surfactant or natural surfactant. Nonionic surfactant is selected from the group consisting of fatty acid glyceride, polyoxyethylene fatty alcohol ethers, polyoxyethylene sorbitan monoacid esters, sorbitol and sorbitan fatty acid ester, polyoxyethylene fatty acid ethers, vitamin E derivatives and polyoxyolefin copolymer; the natural surfactant is selected from the group consisting of egg yolk lecithin, fabaceous lecithin, ornitrol and cholic acids, sodium alginate and chitosan. The preferable for emulsifier is natural surfactant, and the best emulsifier is natural egg yolk lecithin and soybean lecithin. The content of the emulsifier in the submicron emulsion of the present invention is 0.5%-5% (g/ml) of the total submicron emulsion complex, preferably, 1.0%-4.0% (g/ml), and the best is 1.0%-2.0% (g/ml).

In the present invention, the assistant emulsifier as described is selected from the group consisting of polyethyleneglycol (PEG) and poloxamer. The content of the assistant emulsifier is 0%-5% (g/ml) of the total amount of the submicron emulsion in the present invention, preferably 0.5%-3% (g/ml), the best is 1.0%-2.0% (g/ml).

In the present invention, the osmo-regulator (isotonic agent) of the paclitaxel submicron emulsion is selected from the group consisting of glycerin, xylitol, sorbierite and mannitol, preferably glycerin and glucose, best glycerin. The content of glycerin is 1.0-3.0% (g/ml) of the total amount of paclitaxel submicron emulsion in the present invention, preferably 1.5-2.5% (g/ml).

In the present invention, the paclitaxel submicron emulsion as described could add stabilizer, which could be one selected from the group consisting of among oleic acid, eunatrol and PEGS, preferably oleic acid and PEG, best oleic acid. The content of oleic acid is 0.05-0.5% (g/ml) of the total amount of paclitaxel submicron emulsion in the present invention, preferably 0.1-0.2% (g/ml).

The paclitaxel submicron emulsion in the present invention could also include antioxidant, which could be vitamin E or vitamin E ester derivatives, preferably vitamin E.

In the present invention, "one or mixture" or "at least one" means it could be one material being chosen, or a mixture of two or more of them.

Another purpose of the present invention is to provide one preparing procedure for the paclitaxel submicron emulsion as described in the present invention, including the following steps:

> ► Prepare water for injection, add emulsifier, assistant emulsifier and isotonic agent, get an even water phase by dispersing in a tissue disintegrator or shear, heating up to 40-80°C, and insulate;
> ► Get paclitaxel/steroid complex, choose any stabilizer and dissolve both of them into the oil for injection preheated up to 40-80°C, get an even oil phase by dispersing in a tissue disintegrator or shear;
> ► Under agitating, add the oil phase into the water phase slowly, disintegrate for 5-10 min at 10000-20000 r/min to obtain a preliminary emulsion, and transfer it into a high pressure homogenizer quickly, particles with diameter under 400 nm are obtained through homogeneous emulsification, preferably under 300 nm, collect all the emulsion, adjust its pH to 3.5-6.0 through hydrochloric acid, preferably 4.0-5.0, add appropriate amount of water (to full volume), thus the product is obtained.

The present invention also provides another preparing procedure for the paclitaxel submicron emulsion, including the following steps:

> ► Prepare water for injection, add assistant emulsifier and isotonic agent, get a water phase by agitating, heating up to 40-80°C, and insulate;
> ► Get paclitaxel/steroid complex, emulsifier and/or stabilizer, dissolve them into the oil for injection, get an even oil phase by dispersing in a tissue disintegrator or shear;
> ► Under agitating, add the water phase into the oil phase slowly, disintegrate for 5-10 min at 10000-20000 r/min to obtain a preliminary emulsion, and transfer it into a high pressure homogenizer quickly, particles with diameter under 400 nm are obtained through homogeneous emulsification, preferably 100-300 nm, collect all the emulsion, adjust its pH to 3.5-6.0 through hydrochloric acid, preferably 4.0-5.0, add appropriate amount of water (to full volume), thus the product is obtained.

In the preparing procedure of the present invention, emulsification in the high pressure homogenizer could be replaced by other emulsification measures as long as an even emulsification could be performed and the particle diameter as described is realized; the temperature, 40-80°C, could be 40°C, 50°C, 60°C, 70°C or 80°C.

In the preparing procedure of the present invention, the concentration for the hydrochloric acid used to adjusting the pH could be a commonly used in this area, such as 0.1 mol/L or 0.01 mol/L; the amount of water and oil for injection could be determined by technicians in this field according to the ratio of the oil phase provided by the present invention, and technicians in this field could add water according to common specifications to get the amount of water corresponding to the drug loading provided by the present invention.

The present invention also provides a formulation, which includes the paclitaxel submicron emulsion and could be made into any preparation which could be clinically used, including infusion solution or dry emulsion. Wherein the infusion solution is prepared by the following procedure: after filling the paclitaxel submicron emulsion described in the present invention, the aseptic process is performed through circulating steam sterilization or steam sterilization, and the target preparation is obtained; wherein the dry emulsion is prepared by the following procedure: add appropriate amount of support agent into the paclitaxel submicron emulsion described in the present invention, after aseptic process, dry emulsion is obtained through freeze-drying process. The preferred support agent is mannitol, such as 5% (w/v) mannitol.

The present invention also provide the application of the paclitaxel submicron emulsion of the present invention in the preparation of anticancer drugs, and the cancer as described is solid tumor, including oophoroma, breast cancer, cervical carcinoma, non-small cell, head or neck cancer, esophagus cancer, renal carcinoma, liver cancer and gastric cancer.

Unless otherwise specified, all the scientific and technological terminology and name used in the present invention means the same as the understanding of common technicians in the field that the present invention belongs to; furthermore, if not specified, the material and its content or proportion, equipment, instrument, preparing condition are all those that the technicians in this field are familiar with or could understand based on the description in the present invention.

The paclitaxel sumicron emusion provided by the present invention has the following special advantages:

1) Low manufacture cost and steroid intake: the paclitaxel submicron emulsion of the present invention uses a steroid complex with low liposome material as the intermediate carrier, since the amount of steroid in the complex is just 0.09~1.94 of the paclitaxel by weight (preferably 0.11~0.97), thus this patent application greatly reduces liposome material used, manufacture cost and risks caused by high dose of steroids compared to "Paclitaxel Submicron Emulsion Using Liposome as Intermediate Carrier" disclosed in patent application 200810168212.5,

2) Good entrapment efficiency and stability: single dose of clinically used paclitaxel is 135~175 mg/m$^2$, i.e. 240-300 mg a dose per one person. Besides, based on the submicron emulsion volume that human body could accept at one time, the volume of single dose of paclitaxel submicron emulsion should be within the range of **100-500 ml**. Calculated according to these, the concentration of paclitaxel in the submicron emulsion should fall in the range of 0.48 mg/ml-3.0 mg/ml. In "Paclitaxel Submicron Emulsion Using Liposome as Intermediate Carrier" disclosed in 200810168212.5, the submicron emulsion formulation prepared using phospholipid complex as the intermediate carrier, the drug load is 0.5 mg/ml at most, and it is unstable after storage; comparing to this, the submicron emulsion formulation prepared using cholesterol complex as the intermediate carrier hat a higher drug load, however, the amount of cholesterol is up to 1~19 times of paclitaxel, resulting in that the total amount of complex wrapped in paclitaxel submicron emulsion is up to 1.2 mg/ml-60 mg/ml. As for the submicron emulsion formulation using steroid complex as intermediate carrier this patent application, since the amount of steroid used is only 0.09~1.94 of that of paclitaxel (preferably 0.11~0.97), the total amount of complex wrapped in the emulsion is only 1.14 mg/ml-3.60 mg/ml (preferably 1.39 mg/ml-1.80 mg/ml), thus, the formulation in the present invention has superior stability and is rather safe to human body.

As for the oil-in-water submicron emulsion formulation, the lipid soluble drug exists in the oil phase and/or the interface between oil and water, the volume of the oil droplet inside the oil phase and the volume of the interface are limited, the entrapment of the emulsion drops as the total amount of complexes used increase, resulting in that more drug goes into the outer water phase and the stability of submicron emulsion after long-term storage will decrease. Compared to the technology disclosed in 200810168212.5, in this patent, the total amount of the complexes wrapped in the oil phase significantly decrease due to the use of a steroid complex with low liposome material content as the intermediate carrier, in this way, the entrapment efficiency of the submicron emulsion is increased, the free drug in the water phased is reduced and the stability of the formulation is improved.

Comparing studies show that the entrapment efficiency of the submicron emulsion disclosed in 200810168212.5 is between 65%-85%, stratification appears after storage under refrigeration (4°C, the same below) up to 12 months, the content decreases, the impurity increases from preliminary 1% to 3.5%~7.6%; as for the submicron emulsion in this patent application, the entrapment efficiency is above 90%, no stratification is found after storage under refrigeration up to 12 months, the appearance is evenly adularescent, there is no obvious change with regard to the diameter or content of the particles, total impurity is less than 1.7%, within the range of preferable drug load, the entrapment efficiency is above 95%, the total impurity is under 1%, and the quality is stable.

3) Good safety and high tolerated dosage: compared to common solution for injection available, the submicron emulsion provided in the present invention is alcohol and Cremopher EL free, which eases the vascular stimulation of paclitaxel formulation, prevents potential allergic reactions and toxicity and side effects caused by Cremopher EL, thus, the safety is improved and the tolerated dosage is increased, providing a solid basis increasing dosage and improving efficacy. When nude mouse are injected with one dosage every 3 days for 3 times, the maximum tolerated dose (without animal death) for common injection available is 20 mg/kg, while for the submicron emulsion in this patent application is 45 mg/kg, and the tolerated dose increases to 2.25 times and is identical to that of albumin-bound paclitaxel nanoparticle available reported in literature (increasing to 2.23 times of that of common injections). By comparing the anticancer activity under tolerated dose, the result shows that the submicron emulsion in this patent application has highest tumor inhibition rate among breast cancer, oophoroma and lung cancer at a dose of 45 mg/kg if compared to common injection 20 mg/kg and albumin-bound paclitaxel nanoparticle for injection 45 mg/kg, and the tumor growth is significantly slower than normal injection and albumin-bound paclitaxel nanoparticle for injection.

In the following section, the present invention will be further explained referring to description of figures and examples, however, technicians in this field should be aware of that the present invention is not limited to these examples and preparing procedure used. What's more, technicians in this field could perform equivalent substitution, combination, improvement or modification, however, all of these have been included in what is claimed the present invention.

**Description of Figures**

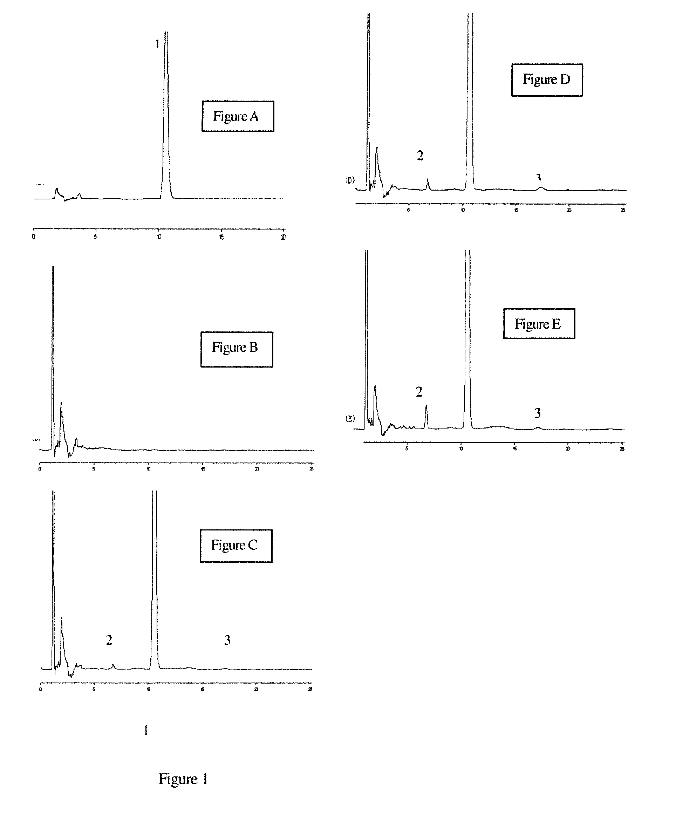

Figure 1: HPLC examination graph of submicron emulsion in experimental example 2 (figure A is paclitaxel control; figure B is blank emulsion; figure C is submicron emulsion 14 preliminary; figure D is submicron emulsion 14 stored at 4°C up to 12 months; figure E is submicron emulsion 31 stored at 4°C up to 12 months. In which, peak 1 is paclitaxel, peak 2~3 are impurities).

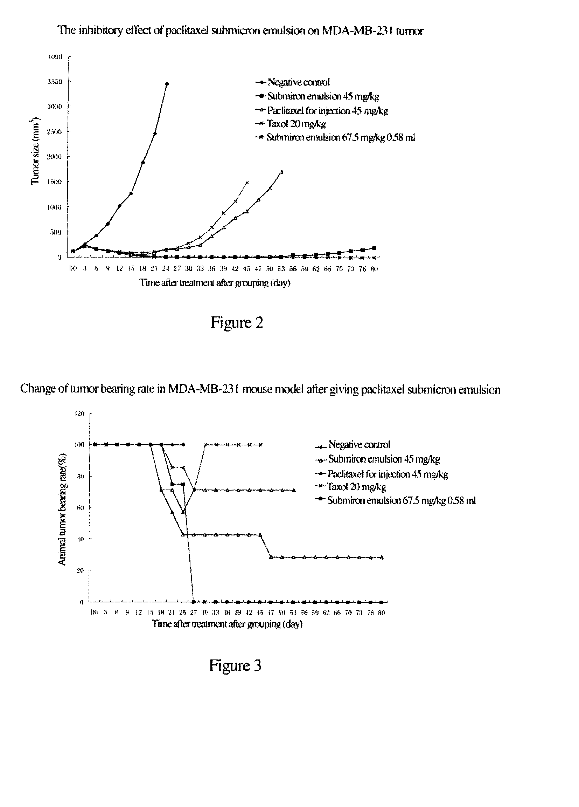

Figure 2: experimental example 6, the inhibitory effect of different formulations on MDA-MB-231 tumor
Figure 3: experimental example 6, change of tumor bearing rate in MDA-MB-231 mouse model after giving different formulations

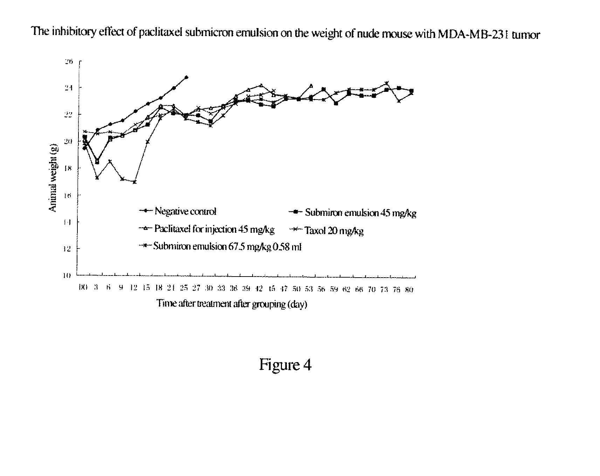

Figure 4: Example 6, the inhibitory effect of different formulations on the weight of nude mouse with MDA-MB-231 tumor

**Examples**

**Example 1 Paclitaxel/ steroid complex**

Test Complex 1~Complex 6: take cholesterol, 7-hydrogenated cholesterol and Ergosterol as Liposome material according to the technical requirements of the invention patent, preparation of 2 Paclitaxel /cholesterol complexs (Molar ratio is 1: 1 and 1:2),2 Paclitaxel /7-hydrogenated cholesterol complexs (Molar ratio is 1: 1 and 1:4),2 Paclitaxel /Ergosterol complexs(Molar ratio is 1: 1 and 1:4). Preparation method: dissolve Paclitaxel and steroid in a flask, by adding 2000ml acetone, with constant stirring gently at 40°C for I hour, combine the washing to the rotary evaporator, remove from solvent, decompressed and dried in vacuum at 40°C for 24 hours.

Reference Complex 1~Reference Complex 4: Using phospholipid and cholesterol as Liposome material according to the technical requirements of complex of patent 200810168212.5, preparation of 2 Paclitaxel /phospholipid complexs (Molar ratio is 1: 6 and 1: 10),2 Paclitaxel /cholesterol complexs (Molar ratio is 1: 10 and 1:20). Its preparation method is the same as Test Complex 1~Complex 6.

Composition and Preparation results of 6 Complexs and 4 Reference Complexes as provided below.

Table 1: Composition of Paclitaxel /steroid complex

| Complex No./Liposome material | Paclitaxel inventory | Liposome material inventory | Paclitaxel /Liposome material | |
|---|---|---|---|---|
| | | | Molar ratio | Weight ratio |
| Test Complex 1/ cholesterol | 8.0 g | 3.6 g | 1:1 | 1:0.45 |
| Test Complex 2/ cholesterol | 8.0 g | 7.2 g | 1:2 | 1:0.90 |
| Test Complex 3/ 7-hydrogenated cholesterol | 3.0 g | 1.35g | 1:1 | 1:0.45 |
| Test Complex 4/ 7-hydrogenated cholesterol | 3.0 g | 5.406g | 1:4 | 1:1.80 |
| Test Complex 5/ Ergosterol | 3.0g | 1.40g | 1:1 | 1:0.46 |
| Test Complex 6/ Ergosterol | 3.0g | 5.58g | 1:4 | 1:1.86 |
| Reference Complex 1/ phospholipid | 3.0 g | 16.65g | 1:6 | 1:5.55 |
| Reference Complex 2/ phospholipid | 3.0 g | 6.78g | 1:10 | 1:9.23 |

(continued)

| Complex No./Liposome material | Paclitaxel inventory | Liposome material inventory | Paclitaxel /Liposome material | |
|---|---|---|---|---|
| | | | Molar ratio | Weight ratio |
| Reference Complex 3/ cholesterol | 3.0g | 13.5g | 1:10 | 1:4.50 |
| Reference Complex 4/ cholesterol | 3.0g | 27.18g | 1:20 | 1:9.06 |

**Example 2: Paclitaxel submicro-emulsion using Paclitaxel cholesterol Complex as intermediate carrier**

**[Composition]**

| Component | submicro-emulsion 1 | submicro-emulsion 2 | submicro-emulsion 3 | submicro-emulsion 4 |
|---|---|---|---|---|
| Test Complex 1* | 145mg | 290mg | 580mg | 1160mg |
| Egg Yolk Lecithin | 2g | 2.4g | 3g | 3g |
| Poloxamer(188) | 1g | 2g | 4g | 6g |
| Glycerol | 5g | 5g | 5g | 5g |
| Soybean oil | 40ml | 40ml | 5ml | 50ml |
| Water for injection added to | 200ml | 200ml | 200ml | 200ml |
| Volume dose | 200ml | 200ml | 200ml | 200ml |
| * Test Complex 1 is the complex prepared by Example 1, weight ratio of Paclitaxel /cholesterol is 1:0.45. | | | | |

**[Preparation method]**

► Disperse the measured Egg Yolk Lecithin , poloxamer(188) and glycerol with 130-140ml water for injection in the blender, stirring to form homogeneous water phase, heat to 40°C,keep warm;
► Heat the measured soybean oil to 40°C,weigh Paclitaxel cholesterol Test Complex I prepared by Example 1, dissolved in soybean oil, stirring to form homogeneous oil phase in the blender;
► The water phase is added slowly to the oil phase under stirring conditions, at the rotation speed of 10000 /min for emulsifying of 5min, and transferred onto a High Pressure Homogenizer and homogenize for 6 times, collect the emulsion, adjust PH value to 4.0±0.5 with 0.1mol/L HCl, and add water to 200ml,and mix well, sterilization for 30 min at 115°C after separate-loading.

Content of Submicron emulsion1-Submicro emulsion4,emulsifying agent (Egg Yolk Lecithin) is 1.0%(g/ml), 1.2%(g/ml),1.5%(g/ml) and 1.5%(g/ml) of the submicro-emulsion respectively, and content of co-emulsifier agent poloxamer(188) is 0.5%(g/ml),1.0%(g/ml),2.0%(g/ml) and 3.0%(g/ml),and drug loading rate of paclitaxel is 0.5mg/ml, 1.0mg/ml,2.0mg/ml,4.0mg/ml respectively. Average particle diameter of 4 groups of emulsion is 225nm,233nm, 245nm23nm respectively determined by laser particle sizer.

**Example 3:Paclitaxel submicro-emulsion using Paclitaxel cholesterol Complex as intermediate carrier**

**[Composition]**

| Component | submicro-emulsion 5 | submicro-emulsion 6 | submicro-emulsion 7 | submicro-emulsion 8 |
|---|---|---|---|---|
| Test Complex 2* | 190mg | 380mg | 760mg | 1520mg |

(continued)

| Component | submicro-emulsion 5 | submicro-emulsion 6 | submicro-emulsion 7 | submicro-emulsion 8 |
|---|---|---|---|---|
| Egg Yolk Lecithin | 2g | 2.4g | 3g | 3g |
| Poloxamer(188) | 2.4g | 4g | 4g | 6g |
| Glycerol | 5g | 5g | 5g | 5g |
| Vitamin E | / | / | / | 40mg |
| Soybean oil | 40ml | 40ml | 50ml | 50ml |
| Water for injection added to | 200ml | 200ml | 200ml | 200ml |
| Volume dose | 200ml | 200ml | 200ml | 200ml |
| * Test Complex 2 is the complex prepared by Example 1, weight ratio of Paclitaxel /cholesterol is 1:0.90. | | | | |

**[Preparation method]**

► Weigh Egg Yolk Lecithin ,poloxamer(188) and glycerol, dissolved in 130-140ml water for injection, stirring to form homogeneous water phase in the blender ,and heat to 40-80°C, keep warm;
► Heat the measured soybean oil to 80°C,weigh Complex 2 Egg Yolk Lecithin and Vitamin E ,dissolved in soybean oil, stirring to form homogeneous oil phase in the blender;
► The water phase is added slowly to the oil phase under stirring conditions, at the rotation speed of 10000/min for emulsifying of 5min, and transferred onto a High Pressure Homogenizer and homogenize for 6 times, collect the emulsion, adjust PH value to 5.5±0.5 with 0.1 mol/L HCl, and add water to 200ml,and mix well, sterilization for 30 min at 115°C after separate-loading.

Content of Submicro emulsion5-Submicro emulsion8,emulsifying agent (Egg Yolk Lecithin) is 1.0%(g/ml),1.2% (g/ml),1.5%(g/ml) and 1.5%(g/ml) of the submicro-emulsion respectively, and content of co-emulsifier agent poloxamer (188) is 1.2%(g/ml),2.0%(g/ml),2.0%(g/ml) and 3.0%(g/ml),and drug loading rate of paclitaxel is 0.5mg/ml,1.0mg/ml, 2.0mg/ml,4.0mg/ml respectively. Average particle diameter of 4 groups of emulsion is 246nm,262nm,231nm and 242nm respectively determined by laser particle sizer.

**Example 4:Paclitaxel submicro-emulsion using Paclitaxel cholesterol Complex as intermediate carrier**

**[Composition]**

| Component | Submicro emulsion9 | Submicro emulsion10 | Submicro emulsion11 | Submicro emulsion12 |
|---|---|---|---|---|
| Test Complex 1* | 145mg | 290mg | 580mg | 1450mg |
| soyabean lecithin | 2.4g | 2.4g | 2.4g | 3.0g |
| poloxamer(188) | 4g | 4g | 4g | 4g |
| Glycerol | 5g | 5g | 5g | 5g |
| Vitamin E | / | / | / | 40mg |
| MCO | 40ml | 40ml | 50ml | 50ml |
| Water for injection added to | 200ml | 200ml | 200ml | 200ml |
| Volume dose | 200ml | 200ml | 200ml | 200ml |
| * Test Complex 1 is the complex prepared by Example 1, weight ratio of paclitaxel /cholesterol is 1:0.45. MCO: medium chain oil | | | | |

**[Preparation method]**

It is It is the same as Example 3 . PH value of Submicro emulsion is adjusted to 5.0±0.5.

Content of emulsion9-Submicro emulsion12,emulsifying agent(soyabean lecithin) is 1.2%(g/ml),1.2%(g/ml), 1.2%(g/ml) and 1.5%(g/ml) of the submicro-emulsion respectively, and content of co-emulsifier agent poloxamer(188) is 2.0%(g/ml) of submicro-emulsion, and drug loading rate of paclitaxel is 0.5mg/ml,1.0mg/ml,2.0mg/ml,5.0mg/ml respectively. Average particle diameter of 4 groups of emulsion is 165nm,153nm,127nm,138nm respectively determined by laser particle sizer.

**Example 5:Paclitaxel submicro-emulsion using Puclitaxel cholesterol Complex as intermediate carrier**

**[Composition]**

| Component | Submicro emulsion13 | Submicro emulsion14 | Submicro emulsion15 | Submicro emulsion16 |
|---|---|---|---|---|
| Test Complex 1* | 290mg | 435mg | 580mg | 1450mg |
| soyabean lecithin | 2.4g | 2.4g | 2.4g | 4.0g |
| poloxamer(188) | 3g | 3g | 4g | 4g |
| Glycerol | 5g | 5g | 5g | 5g |
| Oil mixture** | 40ml | 40ml | 40ml | 50ml |
| Water for injection added to | 200ml | 200ml | 200ml | 200ml |
| Volume dose | 200ml | 200ml | 200ml | 200ml |

* Test Complex 1 is the complex prepared by Example 1, weight ratio of Pactitaxel /cholesterol is 1:0.45.
**Oil mixture is the mixture of soybean oil /MCO(volume ratio 1:1).

**[Preparation method]**

It is the same as Example 3. PH value of Submicro emulsion is adjusted to 4.5±0.5.
Purity of Submicro emulsion13-Submicro emulsion16,emulsifying agent (soyabean lecithin) is 1.2%(g/ml),1.2% (g/ml),1.2%(g/ml) and 2.0%(g/ml) of the submicro-emulsion respectively, and content of co-emulsifier agent poloxamer (188) is 1.5%(g/ml),1.5%(g/ml),2.0%(g/ml) and 2.0%(g/ml),and drug loading rate of paclitaxel is 1.0mg/ml,1.5mg/ml, 2.0mg/ml and 5.0mg/ml respectively. Average particle diameter of 4 groups of emulsion is 145nm,138nm,133nm,146nm respectively determined by laser particle sizer.

**Example 6:Paclitaxel submicro-emulsion using Paclitaxel cholesterol Complex as intermediate carrier**

[Composition]

| Component | Submicro emulsion17 | Submicro emulsion18 | Submicro emulsion19 | Submicro emulsion20 |
|---|---|---|---|---|
| Test Complex 2* | 190mg | 380mg | 760mg | 1520mg |
| Egg Yolk Lecithin | 3.0g | 3.0g | 4.0g | 6.0g |
| poloxamer(188) | 4g | 4g | 6g | 6g |
| Glycerol | 5g | 5g | 5g | 5g |
| Oil mixture** | 30ml | 40ml | 50ml | 60ml |
| Water for injection added to | 200ml | 200ml | 200ml | 200ml |
| Volume dose | 200ml | 200ml | 200ml | 200ml |

*Test Complex 2 is the complex prepared by Example 1, weight ratio of Paclitaxel /cholesterol is 1:0.90.
**Oil mixture is the mixture of soybean oil /MCO(volume ratio 1:1).

**[Preparation method]**

It is the same as Example 2. PH value of Submicro emulsion is adjusted to 5.5±0.5.

Purity of Submicro emulsion17-Submicro emulsion20,emulsifying agent (Egg Yolk Lecithin ) is 1.5%(g/ml), 1.5%(g/ml),2.0%(g/ml) and 3.0%(g/ml) of the submicro-emulsion respectively, and content of co-emulsifier agent poloxamer(188) is 2.0%(g/ml),2.0%(g/ml),3.0%(g/ml) and 3.0%(g/ml),and drug loading rate of paclitaxel is 0.5mg/ml, 1.0mg/ml,2.0mg/ml and 5.0mg/ml respectively. Average particle diameter of 4 groups of emulsion is 255nm,263nm, 285nm,232nm respectively determined by laser particle sizer.

**Example 7:Paclitaxel submicro-emulsion using Paclitaxel cholesterol Complex as intermediate carrier**

**[Composition]**

| Component | Submicro emulsion21 | Submicro emulsion22 | Submicro emulsion23 | Submicro emulsion24 |
|---|---|---|---|---|
| Test Complex 1* | 145mg | 290mg | 580mg | 1160mg |
| Fatty glyceride | 3.0g | 4.0g | / | / |
| Polyoxyethylene-Sorbitan Fatty Acid Ester | / | / | 4.0g | 6.0g |
| poloxamer(188) | 3g | 4g | 4g | 6g |
| Glycerol | 5g | 5g | 5g | 5g |
| Oleic acid | 0.2g | 0.2g | 0.2g | 0.2g |
| Oil mixture** | 30ml | 40ml | 50ml | 50ml |
| Water for injection added to | 200ml | 200ml | 200ml | 200ml |
| Volume dose | 200ml | 200ml | 200ml | 200ml |

*Test Complex I is the complex prepared by Example 1, weight ratio of Paclitaxel /cholesterol is 1:0.45.
**Oil mixture is the mixture of soybean oil /MCO(volume ratio 1:1).

**[Preparation method]**

It is the same as Example 3.
In Submicro emulsion21 and 22,content of fatty glyceride emulsifying agent is 1.5%(g/ml) and 2.0%(g/ml) of the submicro-emulsion, and in Submicro emulsion23 and 24, content of submicro-emulsion Polyoxyethylene Sorbitan Fatty Acid Ester is 2.0%(g/ml) and 3.0%(g/ml) respectively. Content of co-emulsifier agent poloxamer(188) is 1.5%(g/ml), 2.0%(g/ml),2.0%(g/ml) and 3.0%(g/ml) respectively, and drug loading rate of paclitaxel is 0.5mg/ml,1.0mg/ml,2.0mg/ml and 4.0mg/ml respectively. Average particle diameter of 4 groups of emulsion is 145nm,133nm,126nm,158nm respectively determined by laser particle sizer.

**Example 8: Submicro emulsion using 7-hydrogenated cholesterol or Ergosterol complex as intermediate carrier**

**[Composition]**

| Component | Submicro emulsion25 | Submicro emulsion26 | Submicro emulsion27 | Submicro emulsion28 |
|---|---|---|---|---|
| Complex 3* | 290mg | / | / | / |
| Complex 4* | / | 5604mg | / | / |
| Complex 5* | / | / | 292mg | / |
| Complex 6* | / | / | / | 5720mg |
| soyabean lecithin | 2.4g | 2.4g | 2.4g | 4.0g |
| poloxamer(188) | 3g | 3g | 4g | 4g |
| Glycerol | 5g | 5g | 5g | 5g |
| Oil mixture** | 40ml | 40ml | 40ml | 50ml |
| Water for | injection | | | |

(continued)

| Component added to | Submicro emulsion25 200ml | Submicro emulsion26 200ml | Submicro emulsion27 200ml | Submicro emulsion28 200ml |
|---|---|---|---|---|
| Volume dose | 200ml | 200ml | 200ml | 200ml |

*Complex 3-Complex 6 are Paclitaxel /7-hydrogenated cholesterol complex and Paclitaxel /Ergosterol complex prepared by Example I **Oil mixture is the mixture of soybean oil /MCO (volume ratio 1:1).

**[Preparation method]**

The same as Example 5

In submicro emulsion25-Submicro emulsion28, drug loading rate of paclitaxel is 1.0mg/ml. Average particle diameter of 4 groups of emulsion is 143nm,138nm,141nm,132nm respectively determined by laser particle sizer.

**Example 9: Dry emulsion using Paclitaxel cholesterol Complex as intermediate carrier**

Dissolve the 28 groups of Submicro emulsion 50 ml each prepared by Example 2-Example 8 in 3%(w/v) mannitol with constant stirring, and filter with $0.2\mu m$ millipore filter, and dried at a lower temperature.

**Test example**

**Test example 1: Steroid-sparing effect on Entrapment efficiency of submicro-emulsion**

**► Determination method of Entrapment efficiency**

**Determination of total drug content of submicro-emulsion:** Measure accurately 10mL of Emulsion, dilute to volume by adding anhydrous alcohol in a 250mL volumetric flask after demulsification, and mix well as test solution ; Measure accurately some reference Paclitaxel in a flask by adding dehydrated alcohol ,and dilute to $40\mu g/mL$ as test solution. Weigh accurately $20\mu L$ of test solution and reference solution each, and inject in a chromatograph, we use a HPLC method with the column Kromasil-C18(300mm$\times$4.6mm,5$\mu$m),the mobile phase consisted of acetonitrile-water (54:46),the flow rate: 1.0mL/min, the detection wavelength: 230nm,column temperature is at room temperature, sample volume :10$\mu$L, record the chromatograph chart and calculate the peak area and concentration of Paclitaxel and total drug content in the Emulsion by the external standard method, record as W Total.

**Determination of total drug content of in the water phase:** Centrifuge 10 mL of Emulsion on 40,000r/min at16°C for 4.0h,to be layered as oil layer, emulsifying agent layer and aqueous solution layer successively. Record the volume of aqueous solution layer, carefully remove the oil layer and emulsifying agent layer, apply clarified aqueous solution in the bottom ,and filter with 0.22$\mu$m millipore filter, subsequent filtrate is directly sampled, the concentration is determined by the HPLC method as stated above ,and calculate drug content according to volume of the water phase, record as W water phase.

Total drug content of W oil phase and oil-water surface:

$$W\ oil\ phase + oil\text{-}water\ surface = W\ Total - W\ water\ phase,$$

Calculation of entrapment efficiency: Entrapment efficiency $= \dfrac{W\ oil\ phase + oil\text{-}water\ surface}{W\ TOTAL} \times 100\% = \dfrac{(W\ Total - W\ water\ phase)}{W\ TOTAL} \times 100\%$

**► Determination Results of Entrapment efficiency in submicro-emulsion 1~submicro-emulsion 28**

Apply 28 groups of submicro-emulsion prepared by Example 2-Example 7,determined by the HPLC method as stated above and calculate the Entrapment efficiency, the results are shown in Table 2. The results indicated that if content of steroid of the intermediate carrier is 0.09~1.86 of Paclitaxel (take cholesterol as Liposome material, the content is 0.45-0.90 of Paclitaxel), the entrapment efficiency of submicro-emulsion (with different drug loading rates) are above 90%,when Drug loading rate below 2mg/ml,the Entrapment efficiency could reach more than 95%,add less drugs dissociate in the water phase.

Table 2: Determination Results of Entrapment efficiency in 28 groups of submicro-emulsion

| Sample number | Drug loading rate(mg/ml) | Entrapment efficiency |
|---|---|---|
| Submicro emulsion 1 | 0.5 | 96.2% |
| Submicro emulsion2 | 1.0 | 95.1% |
| Submicro emulsion3 | 2.0 | 95.7% |
| Submicro emulsion4 | 3.0 | 93.5% |
| Submicro emulsion5 | 0.5 | 97.7% |
| Submicro emulsion6 | 1.0 | 96.8% |
| Submicro emulsion7 | 2.0 | 96.7% |
| Submicro emulsion8 | 4.0 | 92.6% |
| Submicro emulsion9 | 0.5 | 98.2% |
| Submicro emulsion10 | 1.0 | 98.9% |
| Submicro emulsion11 | 2.0 | 97.6% |
| Submicro emulsion12 | 5.0 | 91.3% |
| Submicro emulsion13 | 1.0 | 98.8% |
| Submicro emulsion14 | 1.5 | 98.1% |
| Submicro emulsion15 | 2.0 | 97.5% |
| Submicro emulsion16 | 5.0 | 90.8% |
| Submicro emulsion17 | 0.5 | 96.5% |
| Submicro emulsion18 | 1.0 | 96.8% |
| Submicro emulsion19 | 2.0 | 97.2% |
| Submicro emulsion20 | 5.0 | 90.5% |
| Submicro emulsion21 | 0.5 | 98.6% |
| Submicro emulsion22 | 1.0 | 97.1% |
| Submicro emulsion23 | 2.0 | 97.2% |
| Submicro emulsion24 | 4.0 | 93.3% |
| Submicro emulsion25 | 1.0 | 98.9% |
| Submicro emulsion26 | 1.0 | 97.1% |
| Submicro emulsion27 | 1.0 | 98.4% |
| Submicro emulsion28 | 1.0 | 98.2% |

► **Preparation of reference submicro-emulsion & Determination Results of Entrapment efficiency**

Apply Reference Complex 1-Reference Complex 4 prepared by Example 1,prepare the submicro-emulsion 29~submicro-emulsion 32 as follows, and the drug loading rate is 0.5,1.0,1.0 and 2.0mg/ml respectively for comparison investigation.

Prescription, Preparation method and measured Entrapment efficiency are as follows:

[Composition]

| Component | Submicro emulsion29 | Submicro emulsion30 | Submicro emulsion31 | Submicro emulsion32 |
|---|---|---|---|---|
| Reference Complex 1* | 655mg | / | / | / |
| Reference Complex 2* | / | 1023mg | / | / |
| Reference Complex 3* | / | / | 1100mg | / |
| Reference Complex 4* | / | / | / | 4024mg |
| Egg Yolk Lecithin | 3g | 3g | 3g | 3g |
| Poloxamer188 | 3g | 3g | 3g | 3g |
| Glycerol | 5g | 5g | 5g | 5g |
| Soybean oil | 40ml | 40ml | 50ml | 50ml |
| Water for injection added to | 200ml | 200ml | 200ml | 200ml |
| Volume dose | 200ml | 200ml | 200ml | 200ml |

*Reference Complex 1,2 are two groups of Reference Complexs prepared by Example 1, weight ratios of Paclitaxel /phospholipid are 1:5.55 and1:9.23 respectively.

*Reference Complex 3,4 are two groups of Reference Complexs prepared by Example 1,weight ratios of cholesterol Paclitaxel /phospholipid are 1:4.50 and 1:9.06 respectively.

## [Preparation method]

Heat the measured soybean oil to 40°C, Paclitaxel cholesterol Test Complex 1 prepared by Example 1, soybean oil, stirring to form homogeneous oil phase in the blender;

Weigh Egg Yolk Lecithin ,poloxamer(188) and glycerol, dissolved in 130-140ml water for injection, stirring to form homogeneous water phase in the blender ,and heat to 40-80°C, keep warm;

Heat the measured soybean oil to 40-80°C, weigh Paclitaxel cholesterol Test Complex 1 prepared by Example 1, dissolved in soybean oil, stirring to form homogeneous oil phase in the blender;

The water phase is added slowly to the oil phase under stirring conditions, at the rotation speed of 10000-20000 /min for emulsifying of 5-10 min, and transferred onto a High Pressure Homogenizer and homogenize for 6 times, collect the emulsion, adjust PH value to $4.5\pm0.5$ with 0.1mol/L HCl, and add water to 200ml,and mix well, sterilization for 30 min at 115°C after separate-loading.

## [Determination of Entrapment efficiency]

Apply submicro-emulsion 29 to submicro-emulsion 32,determine the Entrapment efficiency by the method mentioned above, Entrapment efficiency is 65.7%~84.5%. Please see the Table 3 below.

Table 3: Determination Result of Entrapment efficiency in Reference submicro-emulsion (Submicro emulsion29~Submicro emulsion32)

| Sample of reference Submicro emulsion | Results of Entrapment efficiency |
|---|---|
| Submicro emulsion29 | 75.1% |
| Submicro emulsion30 | 65.7% |
| Submicro emulsion31 | 84.5% |
| Submicro emulsion32 | 83.2% |

## Test example 2: stability study of submicro-emulsion

Apply 28 groups of submicro-emulsion prepared by Example 2-Example 8 (marked as Emulsion1~28 in the following tables) and 4groups of reference submicro-emulsion prepared by Test example 1 (record as Emulsion29~Emulsion32 in the following tables), stored at 4°C for 12 months respectively, samples will be measured at 0,6 and 1.2 month, investigate the differences of appearance, particle diameter, purity and impurity by the following method.

**Character:** visual method, describe the color of submicro-emulsion, record whether there is oil droplets or separation on the surface.

**Particle diameter:** Determine the particle diameter of the submicro-emulsion by MASTER SIZER 2000 laser particle sizer (MALVERN).

Purity and related substances:

Measure accurately defined amount of Paclitaxel submicro-emulsion and add anhydrous alcohol with demulsification method, to prepare test solution with suitable concentration. Weigh accurately 20μL of test solution, inject in a chromatograph, we use a HPLC method with the column Kromasil-C18(300mm×4.6mm,5μm),the mobile phase consisted of acetonitrile-water (54:46),the flow rate: 1.0mL/min, the detection wavelength: 230nm,column temperature is at room temperature, record the chromatograph chart and calculate total drug content of Emulsion according to the peak area by the external standard method, calculate Impurity content by using the normalization method.

**Result:** See the table below.

Table 4 Comparative result of stability among submicro-emulsion

| Sample number/Drug loading rate | The original (determined within a week after preparation) | | | | | Keep for 12 months at 4°C | | |
|---|---|---|---|---|---|---|---|---|
| | Appearance | particle diameter | Purity | Impurity | Uniform | particle diameter | Purity | Impurity |
| Emulsion1/0.5 mg/ml | Uniform | 225nm | 100.2% | 0.33% | Uniform | 221nm | 99.7% | 0.67% |
| Emulsion2/1.0mg /ml | Uniform | 233nm | 97.6% | 0.31% | Uniform | 245nm | 97.3% | 0.62% |
| Emulsion3/2.0mg/ml | Uniform | 245nm | 98.5% | 0.31% | Uniform | 236nm | 98.2% | 0.64% |
| Emulsion4/3.Omg /ml | Uniform | 230nm | 97.6% | 0.37% | Uniform | 237nm | 97.0% | 0.93% |
| Emulsion5/0.5mg /ml | Uniform | 246nm | 99.5% | 0.35% | Uniform | 228nm | 98.8% | 0.52% |
| Emulsion6/1.Omg /ml | Uniform | 262nm | 100.3% | 0.30% | Uniform | 255nm | 99.5% | 0.61% |
| Emulsion7/2.0mg /ml | Uniform | 231nm | 98.1% | 0.36% | Uniform | 240nm | 98.6% | 0.57% |
| Emulsion8/4.Omg /ml | Uniform | 242nm | 99.6% | 0.42% | Uniform | 251nm | 97.3% | 1.01% |
| Emulsion9/0.5mg /ml | Uniform | 165nm | 98.8% | 0.35% | Uniform | 126nm | 99.2% | 0.56% |
| Emulsion10/1.0m g/ml | Uniform | 153nm | 101.6% | 0.30% | Uniform | 133nm | 100.7% | 0.52% |
| Emulsion11 /2.0mg/ml | Uniform | 127nm | 102.2% | 0.32% | Uniform | 131nm | 100.9% | 0.55% |
| Emulsion2/5.0m g/ml | Uniform | 138nm | 99.8% | 0.45% | Uniform | 155nm | 99.4% | 1.24% |
| Emulsion13/1.0m g/ml | Uniform | 145nm | 97.4% | 0.31% | Uniform | 138nm | 98.0% | 0.57% |
| Emulsion14/1.5m g/ml | Uniform | 138nm | 100.3% | 0.35% | Uniform | 136nm | 98.7% | 0.65% |
| Emulsion15 /2.0mg/ml | Uniform | 133nm | 99.2% | 0.31% | Uniform | 137nm | 98.5% | 0.63% |
| Emulsion16/5.0m g/ml | Uniform | 146nm | 98.5% | 0.43% | Uniform | 173nm | 97.2% | 1.68% |
| Emulsion17 /0.5mg/ml | Uniform | 255nm | 97.8% | 0.32% | Uniform | 244nm | 97.6% | 0.57% |
| Emulsion18/1.0m g/ml | Uniform | 263nm | 99.2% | 0.36% | Uniform | 259nm | 99.5% | 0.53% |
| Emulsion19/2.0m g/ml | Uniform | 285nm | 100.4% | 0.33% | Uniform | 272nm | 98.8% | 0.61% |
| Emulsion20/5.0m g/ml | Uniform | 232nm | 101.4% | 0.48% | Uniform | 258nm | 97.8% | 1.76% |
| Emulsion21/0.5m g/ml | Uniform | 145nm | 98.7% | 0.36% | Uniform | 136nm | 99.1% | 0.59% |
| Emulsion22 /1.0mg/ml | Uniform | 133nm | 98.2% | 0.32% | Uniform | 127nm | 97.6% | 0.57% |
| Emulsion23/2.0m g/ml | Uniform | 126nm | 101.1% | 0.38% | Uniform | 134nm | 99.5% | 0.56% |
| Emulsion24/4.0m g/ml | Uniform | 158nm | 98.8% | 0.41% | Uniform | 163nm | 97.9% | 1.25% |

EP 2 494 957 A1

EP 2 494 957 A1

(continued)

| Sample number/Drug loading rate | The original (determined within a week after preparation) | | | | | Keep for 12 months at 4°C | | |
|---|---|---|---|---|---|---|---|---|
| | Appearance | particle diameter | Purity | Impurity | Uniform | particle diameter | Purity | Impurity |
| Emulsion25/1.0m g/ml | Uniform | 143nm | 99.5% | 0.33% | Uniform | 128nm | 98.9% | 0.56% |
| Emulsion26/1.0m g/ml | Uniform | 138nm | 97.8% | 0.35% | Uniform | 132nm | 97.4% | 0.58% |
| Emulsion27/1.0m g/ml | Uniform | 141nm | 99.4% | 0.30% | Uniform | 145nm, | 99.1% | 0.61% |
| Emulsion28/1.0m g/ml | Uniform | 132nm | 98.6% | 0.36% | Uniform | 126nm | 98.2% | 0.54% |
| Emulsion29/0.5m g/ml | Uniform | 253nm | 97.5% | 0.92% | layered and floating oil | 323nm | 90.8% | 7.63% |
| Emulsion30/1.0m g/ml | Layered | 522nm | 93.2% | 6.27% | oil-water separation | Untested | Untested | Untested |
| Emulsion31/1.0m g/ml | Uniform | 247nm | 98.6% | 0.62% | Uniform | 263nm | 94.7% | 3.58% |
| Emulsion32/2.0m g/ml | Uniform | 266nm | 98.9% | 0.78% | Slightly layered | 311nm | 92.8% | 4.64% |

Keep the Submicro emulsion~Submicro emulsion28 prepared by the steroid complex as the intermediate carrier of the invention in the refrigerator (4°C) for 12 months, and compare with the original ,1) The average particle diameter of Emulsion with drug loading rate 5.0mg/ml with a trend of ascend, but there is no layer, and no obvious changes in appearance and purity, impurity has increased by 2.0%; 2) The average particle diameter of Emulsion with drug loading rate 4.0mg/ml with a trend of ascend, but there is no layer, and no obvious changes in appearance and purity, impurity has increased buy1.3%;3) The average particle diameter of Emulsion with drug loading rate 3.0mg/ml, there is no layer, and no obvious changes in appearance and purity, impurity is not up tol.0%;4) The average particle diameter of Emulsion with drug loading rate 2.0mg/ml or below, there is no layer, and no obvious changes in appearance and purity, impurity is not up to 0.7%.

I)Keep the emulsion prepared by the reference Paclitaxel /phospholipid complex as the intermediate carrier for 6 months forming uniform emulsion (submicro-emulsion 29) with the drug loading rate 0.5mg/ml, there is no layer, and no obvious changes in appearance and purity, impurity has increased by3.0%. but keep for 12 months, the particle diameter has grown remarkably and impurity has increased above 7%, purity has decreased, and it is layered and floating oil ;2) When the drug loading rises to 1.0mg/ml. it is impossible to form uniform Emulsion (submicro-emulsion 30), crystallizing and oil droplets were observed by in the beginning. Keep the emulsion prepared by the reference Paclitaxel /cholesterol complex as the intermediate carrier forming uniform emulsion (submicro-emulsion 31~32) with the drug loading ratel.0mg/ml and 2.0mg/ml 1) for 6 months, there is no layer, and no obvious changes in appearance and purity in 2 groups with different drug loading rates, impurity has increased by 1.5%;2) for 12 months, the particle diameters of 2 groups of Emulsion have increased, purities have defined, and Impurities rise to 3.58% and 4.64%respectively, when the Drug loading rate rises to 2.0mg/ml, Emulsion is slightly layered.

**Test example 3: Sensitization Test on Paclitaxel submicro-emulsion**

**Test drugs:**

Tested drug solution: Submicro-emulsion 14 prepared by Example 5;

Reference tablet solution: commercial paclitaxel injection "Rhodoxanthin" (5ml: 30mg) diluted with normal saline to solution with concentration of Paclitaxel 2mg/ml before use;

Positive drug solution: 1.0% ovalbumin;

Blank Emulsion solution: prepared by composition of submicro-emulsion 14 in Example 5 without

Paclitaxel cholesterol Complex;

Blank solvent: Mix the Cremophor EL and Dehydrated Alcohol byl:l.(v/v) (simulated composition of commercial paclitaxel injection, and without Paclitaxel,), diluted with normal saline by 3 times before use;

**Laboratory Animal:**

Guinea-pigs (300g±20g, bisexual each half)

**Methods:**

30 guinea-pigs (300g±20g) were randomly divided into 5 groups, 6 in each group, bisexual each half. The guinea-pigs were kept for 1 week before test to observe their activities. Each group of guinea-pigs were given intraperitoneal injection of tested drug solution (submicro-emulsion group), Reference tablet solution (Reference tablet group), positive control solution (positive control group),Blank Emulsion (Blank Emulsion group) and Blank solvent (Blank solvent group) at a dose of 0.3ml each, on every other day for 3 times and were sensitized, test solution group were of Paclitaxel at 2mg/kg; each groups were given intravenous injection of Test drug solution, Reference tablet solution, Positive drug solution, Blank Emulsion and Blank solvent, at a dose of 1.0ml each at post — injection 12d and were sensitized, test solution group were dosed of Paclitaxel at 6mg/kg. Symptoms of each group of guinea-pigs were observed after intravenous injection, results are shown in the table below.

Table 5: Sensitization Study of Paclitaxel Submicro emulsion

| Groups | Symptoms | Results |
|---|---|---|
| 1)Reference tablet group | Scratching the nose, tremble, bristling, tachypnea , gait disturbance, dyspnea ,spasm, rotation ,myasthenia of limbs | Strongly Positive |
| 2)Submicro-emulsion group | Normal | negative |

(continued)

| Groups | Symptoms | Results |
|---|---|---|
| 3) Blank solvent group | Gait disturbance, dyspnea, spasm, rotation ,myasthenia of limbs | Strongly Positive |
| 4)Blank Emulsion group | Normal | negative |
| 5)Positive control group | Scratching the nose, tachypnea ,dejection, gait disturbance, wheeze, spasm, rotation | Strongly Positive |

The results show that, commercial Paclitaxel injection group and blank solvent group had strong allergy effect, while Paclitaxel submicro-emulsion and blank emulsion group had no obvious allergy effect.

**Test example 4: Evaluate the acute toxicity of Paclitaxel submicro-emulsion**

**Test samples:** Submicro emulsion solution, apply Submicro-emulsion 14 prepared by Example 5; Reference tablet solution: commercial Paclitaxel injection with Cremophor EL (5ml:30mg), diluted with normal saline to solution with concentration of Paclitaxel 2mg/ml before use;

**Laboratory Animal:** 70 Kunming mice,♀, 20±2g( Institute of laboratory animal of Chinese academy medical sciences).

**Methods:** 70 Kunming mice were randomly divided into 7 groups, 10 in each group according to their weight, injection administrations are as follows:

Group 1: Reference tablet solution, were injected to have an initial dose at 25mg/kg;
Group 2:Reference tablet solution, were injected to have an initial dose at 29mg/kg;
Group 3:Reference tablet solution, were injected to have an initial dose at 25mg/kg every 4 days.
Group 4:Submicro emulsion solution, were injected to have an initial dose at 29mg/kg for 2 days in succession;
Group 5:Submicro emulsion solution, were injected to have an initial dose at 29mg/kg for 3 days in succession;
Group 6:Submicro emulsion solution, were injected to have an initial dose at 25mg/kg every 4 days;
Group 7: Submicro emulsion solution, were injected to have an initial dose at 44mg/kg.

Results of acute toxicity experience are shown in the following table, please see sketch2 for weight changing curve.

Table 6 Results of acute toxicity experience

| Drug | Symptoms | Results |
|---|---|---|
| 1)Reference tablet (Group 1-Group 3) | Jumping for several times, dyspnea or apnea, fatigue, eye closure, straddle fixedly, tic and tremble (some of them). It was remitted in 5 ~ 20min, but there were still symptoms of astasia and gait disturbance and lasted for several hours. Weight loss and turned darker some days after injection | Group (1): 1 mouse died. Group (2) I mouse died. Group (3) 2 mice died. |
| 2)Submicro-emulsion (Group 4-Group 7) | No obvious immediate symptoms. Weight loss some days after injection, significantly related to doses. Colors of the mice with high dose turned darker | No death in each group. |

The results of Group 1, Group 2 and Group 3 show that, acute toxicity reactions appeared after injection of Reference tablet, there is 1 mouse died each in Group I and Group 2, 2 mice died in Group. No obvious immediate symptoms and death in Group 4-Group 7.

Group 1 and Group 7 were injected at a single dose, but the dose in Group 7 is 1.765 times of Group 1, and toxicity of extraction in Group 7 is lower than Group 1, which indicates that Submicro emulsion prepared by Paclitaxel with lower toxicity and more tolerated dose than the Reference tablet (commercial injection).

**Test example 5: Maximum tolerated dose (MTD) test of Paclitaxel submicro-emulsion**

**Laboratory Animal:** nude mice

**Test sample:**

Commercial injection with Cremophor EL, divided into 3 groups at a doss of 20, 30 and 45mg/kg respectively;

Submicro-emulsion 14prepared by Example 5, divided into 4 groups at a doss of 30, 45, 67.5 and 101.25 mg/kg respectively;

**Methods:** Intravenous injection at an initial dose every 4 days and for 3 times. Observe obvious immediate symptoms and death from pre-injection to a week after post-injection, record the doss (without death) as maximum tolerated dose (MTD), Results

Table 7: MTD Results of nude mouse

| Test solution | Dose(mg/kg) | Numbers of Animal | Deaths | MTD |
|---|---|---|---|---|
| Ordinary injection | 20 | 10 | 0 | 20mg/kg |
| | 30 | 10 | 1 | |
| | 45 | 5 | 3 | |
| Submicro-emulsion | 30 | 10 | 0 | 45 mg/kg |
| | 45 | 10 | 0 | |
| | 67.5 | 8 | 1 | |
| | 101.25 | 8 | 2 | |

MTD of Ordinary injection is 20mg/kg, and MTD of submicro-emulsion is 45mg/kg. By contrast, MTD of Submicro emulsion rises to 2.25 times, which is similar to Abraxane (Protein-bound paclitaxel ) reported in literatures.

**Test example 6: The inhibition of tumor of Paclitaxel submicro-emulsion**

**Purpose:** Observe the inhibition of Paclitaxel submicro-emulsion on human breast carcinoma MDA-MB-23 xenografts in nude mice at the maximum tolerated dose, then compare with Abraxane (Protein-bound paclitaxel ) and commercial injection (Paclitaxel).

**Test samples:**

1) Submicro-emulsion 14 prepared by Example 5;
2) Commercial Paclitaxel injection (Paclitaxel, Beijing Union pharmaceutical factory), batch number 100102. 30mg/ 5ml;
3) Abraxane (Protein-bound paclitaxel ) (Display as Abraxane), product of American Pharmaceutical Partners, batch number 205133, 100mgl/1g;

**Groups:**

Negative control group. Paclitaxel injection group, Abraxane group, and Paclitaxel Submicro emulsion group Paclitaxel injection group, at a doss of 20mg/kg/ once,

Paclitaxel submicro-emulsion group, at a doss of 45mg/kg/ once and 67.5mg/kg/ once,

Abraxane group, at a doss of 45mg/kg/once.

**Tumor model:**

Human breast carcinoma MDA-MB-23 xenografts in nude mice,

Tumor-bearing mouse is from crown Bioscience Inc. (Beijing)

**Experiment process and observation methods:**

The healthy nude mice with MDA-MB-23 xenografts were sacrificed by cervical dislocation. Tissues of tumor were extracted in aseptic conditions, choose some good tissues and cut into blocks dir.2-3mm with the scalpel, inoculated subcutaneously into the nude mice. And tumor grew naturally after inoculation.

Tumor-bearing mice were divided by tumor volume, 7 in each group when the tumors grew to 110~120 mm$^3$. Other groups of mice were given tail vein injection once every 4 days except Negative control group. (Paclitaxel Submicro emulsion (67.5mg/kg) group were given intravenous injection half of volume dose and another half in 1-2h later). Each group of mice injected with intermittent administration for 3 times.

Record the first dose day as (**D0**), and detect changes of the tumors and weight of mice every 3 days while carrying out the experiment.

The 25th day (**D25**), after 3 times of injection, tumors growth were inhibited, when test values of Tumor volumes were equal to or less than starting injection, mice in Negative control group with larger tumors were sacrificed and tumor growth of other groups remains to be observed.

**The 45th day (D45),** the group of mice with Paclitaxel injection 20mg/kg were sacrificed on D45 for the average Tumor volume was close to 1500mm$^3$.

**The 53rd day (D53),** the group of mice with Abraxane injection 45mg/kg were sacrificed on D53 for the average Tumor volume was up to 1700mm$^3$.

**The 80th day(D80),** By the time you finish testing, the average Tumor volume of the mice with Paclitaxel submicroemulsion injection 45mg/kg is 198mm$^3$, and in 67.5mg/kg group the tumor disappeared, and there was no recurrence.

**Conclusion:** In conclusion, on a condition of MTD, anti-tumor rates of the 3 (commercial Paclitaxel Ordinary injection20mg/kg, Paclitaxel submico-emulsian 45mg/kg and Abraxane 45mg/kg) were above 98%, but the inhibitory effect of the 3 groups showed significant difference.

On the 25th day, the relative Tumor volume of Piclitaxel Ordinary injection group and Abraxane group is 1.41 and 0.92 respectively, and Tumor in Paclitaxel submicro-emulsion group almost disappeared and RTV is just 0.06.

On the 45th day, Tumor in ordinary injection group grew fast, RTV was up to 14.3(were sacrificed); Tumor in Abraxane group grew slower than ordinary injection group, but RTV was up to 6.5; and there is almost no increase of Tumor volume in Paclitaxel submicro-emulsion group and RTV is 0.11.

On the 53rd day, RTV of Abraxane group was reached to 12.5 ((were sacrificed), while in Paclitaxel submicro-emulsion group is 0.26.

On the 80th day, RTV of Paclitaxel submicro-emulsion group is 1.83. And the mice in this group were sacrificed for slow growth of Tumor and finish testing.

Table 8: Tumor volume and Tumor condition on the 25th day (D25)

| Groups | Tumor volume (mm$^3$) Primary | D25 | RTV | T/C (%) | Tumor condition tumor/ Mouse Tumor rate | |
|---|---|---|---|---|---|---|
| Negative control group | 110±62.4 | 3452±1080.8 | 37.90±17.32 | | 7/7 | 100% |
| Ordinary injection (Paclitaxel) 20mg/kg | 119±55.7 | 165±192.2 | 1.41±1.170 | 3.72 | 7/7 | 100% |
| BSANP for injection 45mg/kg | 114±32.3 | 158±178.4 | 0.92±1.102 | 2.42 | 5/7 | 71.4% |
| Paclituxel submicro-emulsion 45mg/kg | 116±61.0 | 8±13.8 | 0.06±0.074 | 0.15 | 5/7 | 71.4% |
| 67.5mg/kg | 1122±60.1 | 6±4.8 | 0.08±0.063 | 0.21 | 4/7-3* | 100% |
| *Note: there are 3 mice died in this group during administration, and left 4. Following is the same. | | | | | | |

Table 9: Tumor volume and Tumor condition on the 45th day (D45)

| Group | Tumor volume (mm$^3$) Primary | D45 | RTV | Tumor condition tumor/Mouse Tumor rate | |
|---|---|---|---|---|---|
| Ordinary injection(Paclitaxel) | 119±:55.7 | 1491±1305.3 | 14.34±12.26 | 7/7 | 100% |

(continued)

| Group | Tumor volume (mm$^3$) Primary | D45 | RTV | Tumor condition tumor/Mouse | Tumor rate |
|---|---|---|---|---|---|
| 20mg/kg | | | | | |
| BSANP for Injection 45mg/kg | 114±32.3 | 917±873.4 | 6.53±5.730 | 5/7 | 71.4% |
| Paclitaxel submicro-emulsion 45mg/kg | 116±61.0 | 12±14.2 | 0.11±0.154 | 3/7 | 42.9% |
| 67.5mg/kg | 1122±60.1 | 0 | 0 | 0/7-3* | 0 |

Table 10: Tumor volume and Tumor condition on the 53rd day (D53)

| Group | Tumor volume (mm$^3$) Primary | D45 | RTV | Tumor condition tumor/Mouse-borne Tumor rate | |
|---|---|---|---|---|---|
| BSANP for Injection 45mg/kg | 114±32.3 | 1706±1499.4 | 12.51±10.745 | 5/7 | 71.4% |
| Paclitaxel submicro-emulsion | | | | | |
| 45mg/kg | 116±61.0 | 23±43.6 | 0.26±0.582 | 2/7 | 28.6% |
| 67.5mg/kg | 1122±60.1 | 0 | 0 | 0/7-3* | 0 |

Table 11: Tumor volume and Tumor condition on the 80$^{th}$ day (D80)

| Group | Tumor volume (mm$^3$) Primary | D80 | RTV | Tumor condition tumor/Mouse-borne Tumor rate | |
|---|---|---|---|---|---|
| Paclitaxel Submicro-emulsion Group 45mg/kg | 116±61.0 | 198±317.1 | 1.83±3.624 | 2/7 | 28.6% |
| 67.5mg/kg | 1122±60.1 | 0 | 0 | 0/7-3 | 0 |

**Claims**

1. A paclitaxel submicron emulsion, **characterized in that**,which comprises paclitaxel/steroid complex, oil for injection, water for injection, emulsifier, assistant emulsifier and isotonic agent, in the said paclitaxel/steroid complex, the mole ratio between paclitaxel and steroid is 1:0.2~4; preferably 1:0.2~2; best 1:0.33~1.

2. The paclitaxel Submicron emulsion according to any one of Claims 1~3, **characterized in that**, the said steroid in the paclitaxel/steroid complex is at least one of the natural steroids or their derivatives.

3. The paclitaxel submicron emulsion according to Claim 4, wherein the said natural steroid is is selected from the group consisting of cholesterol, 7-hydrocholesterol, lanosterol, sitosterol, brassicasterol, mycosterol, ostreasterol, stigmasterol, sitosterolum and ergosterol; the said natural steroid derivative is selected from the group consisting of cholic acid, deoxycholic acid and anthropodesoxycholic acid.

4. The submicron emulsion according to any one of Claims 1~3, **characterized in that**, the average droplet diameter of the said submicron emulsion is under 400 nm, the ratio of said oil phase is 5%~35% (ml/ml), the drug load is 0.25 mg/m)~5 mg/ml, preferably 0.5 mg/ml~5mg/ml if measured by paclitaxel; preferably, the average particle diameter of submicron emulsion droplet is under 300 nm, the ratio of said oil phase is 10%~30% (ml/ml), the drug load is 0.5 mg/ml~2 mg/ml, preferably 0.5 mg/ml~5mg/ml if measured by paclitaxel.

5. The submicron emulsion according to Claim 1~4, **characterized in that**, the said oil for injection is one or mixture from long chain or medium chain oil; the said long chain oil is selected from the group consisting of long chain fatty acid, long chain fatty ester or long chain fatty alcohol; the medium chain oil is selected from the group consisting of medium chain fatty acid, medium chain fatty ester and medium chain fatty alcohol.

6. The submicron emulsion according to Claim 5, **characterized in that**, the oil for injection is long chain fatty ester, the said medium chain oil is medium chain fatty acid glyceride.

7. The Submicron emulsion according to Claim 1~4, **characterized in that**, the said emulsifier is nonionic surfactant or natural surfactant, the said nonionic surfactant is selected from the group consisting of fatty acid glyceride, polyoxyethylene fatty alcohol ethers, polyoxyethylene sorbitan monoacid esters, sorbitol and sorbitan fatty acid ester, polyoxyethylene fatty acid ethers, vitamin E derivatives and polyoxyolefin copolymer; the said natural surfactant is selected from the group consisting of egg yolk lecithin, fabaceous lecithin, ornitrol and cholic acids, sodium alginate and chitosan, preferably egg yolk lecithin and soybean lecithi.

8. The submicron emulsion according to Claim 7, **characterized in that**, the content of the said emulsifier is 0%-5% (g/ml) of the total amount of the said submicron emulsion, preferably 1.0%-4.0% (g/ml), the best 1.0%-2.0% (g/ml).

9. The submicron emulsion according to Claim 1~4, **characterized in that**, the said assistant emulsifier is selected from the group consisting of polyethyleneglycol (PEG) and poloxamer 188, preferably poloxamer 188.

10. The submicron emulsion according to Claim 9, **characterized in that**, the content of the said assistant emulsifier is 0%-5% (g/ml) of the total amount of the said submicron emulsion, preferably 0.5%-3% (g/ml), best 1.0%-2.0% (g/ml).

11. The submicron emulsion according to Claim 1~4, **characterized in that**, the said isotonic agent is selected from the group consisting of glycerin, xylitol, sorbierite and mannitol, preferably glycerin.

12. The submicron emulsion of any one of Claims 1~11, **characterized in that**, a stabilizer could also be included and a said stabilizer is selected from the group consisting of oleic acid, eunatrol and PEGS, preferably oleic acid.

13. The submicron emulsion of any one of Claims 1~12, **characterized in that**, and antioxidant could also be included and the said antioxidant is selected from the group consisting of vitamin E or vitamin E ester derivatives, preferably vitamin E.

14. The preparation procedure of submicron emulsion of any one of Claims 1~13, **characterized in that**, comprises the following steps:

   ► Prepare water for injection, add emulsifier, assistant emulsifier and isotonic agent, get an even water phase by dispersing in a tissue disintegrator or shear, heating up to 40-80°C, and insulate;
   ► Mix paclitaxel and steroid at ratio as described, add appropriate volume of organic solvent, agitate under proper temperature, remove the organic solvent, and after vacuum drying, the complex is obtained. Get paclitaxel cholesterol complex, choose any stabilizer and dissolve both of them into the oil for injection preheated up to 40-80°C, get an even oil phase by dispersing in a tissue disintegrator or shear;
   ► Under agitating, add the water phase into the oil phase slowly, disintegrate for 5-10 min at 10000-20000 r/min to obtain a preliminary emulsion, and transfer it into a high pressure homogenizer quickly, particles with diameter under 400 nm are obtained through homogeneous emulsification, preferably under 300 nm, collect all the emulsion, adjust its pH to 3.5-6.0 with hydrochloric acid, preferably 4.0-5.0, add appropriate amount of water, thus the product is obtained.

15. The preparation procedure of submicron emulsion of any one of Claims 1~13, **characterized in that**, comprises the following steps:

   ► Prepare water for injection, add assistant emulsifier and isotonic agent, get a water phase by agitating, heating up to 40-80°C, and insulate;
   ► Mix paclitaxel and steroid at ratio as described, add appropriate volume of organic solvent, agitate under proper temperature, remove the organic solvent, and after vacuum drying, the complex is obtained. Get paclitaxel/steroid complex and emulsifier, dissolve into the oil for injection preheated up to 40-80°C, get an even oil phase by dispersing in a tissue disintegrator or shear;

► Under agitating, add the water phase into the oil phase slowly, agitate and disintegrate at high speed to obtain a preliminary emulsion, and transfer it into a high pressure homogenize quickly, particles with diameter under 400 nm are obtained through homogeneous emulsification, preferably 100-300 nm, collect all the emulsion, adjust its pH to 3.5-6.0 with hydrochloric acid, preferably 4.0-5.0, add appropriate amount of water, thus the product is obtained.

16. A formulation, which comprises paclitaxel submicron emulsion of any one of Claims 1~13 and is infusion solution or dry emulsion.

17. The preparation procedure of the formation according to Claim 16, **characterized in that**, the infusion solution is prepared by the following procedure: after filling the paclitaxel submicron emulsion of any one of Claims 1~13, the aseptic process is performer through circulating steam sterilization or steam sterilization, the preparation is obtained; wherein the dry emulsion is prepared by the following procedure: add appropriate amount of support agent into the paclitaxel Submicron emulsion of any one of Claims 1~13, after aseptic filtering, dry emulsion is obtained through freeze-drying process.

18. Use of the paclitaxel submicron emulsion according to any one of Claims 1~13 or the formulation according to Claim 16 in preparing drugs treating oophoroma, breast cancer, cervical carcinoma, non-small cell, head or neck cancer, esophagus cancer, renal carcinoma, liver cancer and gastric cancer.

19. Use of the formulation according to Claim 16 in preparing drugs treating oophoroma, breast cancer, cervical carcinoma, non-small cell, head or neck cancer, esophagus cancer, renal carcinoma, liver cancer and gastric cancer.

Figure 1

The inhibitory effect of paclitaxel submicron emulsion on MDA-MB-231 tumor

Figure 2

Change of tumor bearing rate in MDA-MB-231 mouse model after giving paclitaxel submicron emulsion

Figure 3

The inhibitory effect of paclitaxel submicron emulsion on the weight of nude mouse with MDA-MB-231 tumor

Figure 4

| | | |
|---|---|---|
| **INTERNATIONAL SEARCH REPORT** | International application No. | |
| | PCT/CN2010/078209 | |

**A. CLASSIFICATION OF SUBJECT MATTER**

See extra sheet
According to International Patent Classification (IPC) or to both national classification and IPC

**B. FIELDS SEARCHED**

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

DATABASE: WPI, EPODOC, CNPAT, CNKI, CA, MEDLINE, EMBASE,STN;

SEARCH TERMS: paclitaxel, taxol, docetaxel, taxane+, taxinol, taxotere, emulsion, cream, microemulsion, submicroemulsion, submicro emulsion, submicron emulsion, suspension, steroid, sterol, cholic acid, oil, emulsifier, emulsifying agent, coemulsifier, isotonizing agent

**C. DOCUMENTS CONSIDERED TO BE RELEVANT**

| Category* | Citation of document, with indication, where appropriate, of the relevant passages | Relevant to claim No. |
|---|---|---|
| X | CN101396343A (INST MEDICAL MATERIALS CHINESE ACAD MEDI), 01 April 2009 (01.04.2009), see examples 2, 4, page 3 paragraph 2, page 4 paragraphs 2-3 | 1-19 |
| A | CN101396346A (INST MEDICAL MATERIALS CHINESE ACAD MEDI), 01 April 2009 (01.04.2009), see example 3 | 1-19 |

☐ Further documents are listed in the continuation of Box C.    ☒ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim (S) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&"document member of the same patent family |

| | |
|---|---|
| Date of the actual completion of the international search | Date of mailing of the international search report |
| 24 January 2011(24.01.2011) | **10 Feb. 2011 (10.02.2011)** |
| Name and mailing address of the ISA/CN<br>The State Intellectual Property Office, the P.R.China<br>6 Xitucheng Rd., Jimen Bridge, Haidian District, Beijing, China<br>100088<br>Facsimile No. 86-10-62019451 | Authorized officer<br>LIANG, Jingchen<br>Telephone No. (86-10)62412201 |

Form PCT/ISA /210 (second sheet) (July 2009)

| INTERNATIONAL SEARCH REPORT | | International application No. | |
|---|---|---|---|
| Information on patent family members | | PCT/CN2010/078209 | |
| Patent Documents referred in the Report | Publication Date | Patent Family | Publication Date |
| CN101396343A | 01.04.2009 | None | |
| CN101396346A | 01.04.2009 | None | |

Form PCT/ISA /210 (patent family annex) (July 2009)

**INTERNATIONAL SEARCH REPORT**

International application No.

PCT/CN2010/078209

Continuation of classification of subject matter:

A61K 9/107 (2006. 01) i

A61K 31/337 (2006. 01) i

A61P 35/00 (2006. 01) i

Form PCT/ISA /210 (extra sheet) (July 2009)

## REFERENCES CITED IN THE DESCRIPTION

**Patent documents cited in the description**

- WO 200810168213X A

- WO 200810168212 A